# EUROPEAN PATENT SPECIFICATION

(11) **EP 3 011 049 B1**
(45) Date of publication and mention of the grant of the patent: **04.04.2018**
(21) Application number: 14736992.0
(22) Date of filing: 16.06.2014
(51) Int. Cl.: C12Q 1/68

(54) **A METHOD AND KIT FOR FOOD ANALYSIS**
VERFAHREN UND KIT ZUR ANALYSE VON NAHRUNG
PROCÉDÉ ET TROUSSE POUR L'ANALYSE ALIMENTAIRE

(30) Priority: 18.06.2013 IT TO20130501
(43) Date of publication of application: 27.04.2016
(73) Proprietor: Consiglio Nazionale Delle Ricerche, 00185 Roma (IT)
(72) Inventor: BREVIARIO, Diego, I-20131 Milano (IT); GIANI', Silvia, I-20131 Milano (IT); MORELLO, Laura, I-20154 Milano (IT); MASTROMAURO, Francesco, I-20134 Milano (IT); GAVAZZI, Floriana, I-23801 Calolziocorte (Lecco) (IT); BRAGLIA, Luca, I-26900 Lodi (IT); PONZONI, Elena, I-24033 Calusco d'Adda (Bergamo) (IT)
(74) Representative: Comoglio, Elena
(86) International application number: PCT/IB2014/062265
(87) International publication number: WO 2014/203148

(56) References cited:
- WO-A1-02/077278
- WO-A1-2011/095860
- WO-A2-01/90413
- WO-A2-2006/050945
- WO-A2-2008/056325
- RASTOGI ET AL: "Species identification and authentication of tissues of animal origin using mitochondrial and nuclear markers", MEAT SCIENCE, ELSEVIER SCIENCE, GB, vol. 76, no. 4, 27 April 2007 (2007-04-27) , pages 666-674, XP022050644, ISSN: 0309-1740, DOI: 10.1016/J.MEATSCI.2007.02.006
- RENÉ CR KÖPPEL ET AL: "Multiplex real-time PCR for the detection and quantification of DNA from beef, pork, horse and sheep", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 232, no. 1, 15 October 2010 (2010-10-15), pages 151-155, XP019855870, ISSN: 1438-2385, DOI: 10.1007/S00217-010-1371-Y

### SEQUENCE LISTING

<110> Consiglio Nazionale delle Ricerche
<120> A method and kit for food analysis
<130> PC1311EC
<160> 3
<170> PatentIn version 3.3
<210> 1
   <211> 22
   <212> DNA
   <213> artificial sequence
<220>
   <223> Primer forward "Afex3Mod"
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (18)..(18)
   <223> n is a, c, g, or t
<400> 1
   gayytdgarc cnggnacnat gg 22
<210> 2
   <211> 19
   <212> DNA
   <213> artificial sequence
<220>
   <223> Primer reverse "Arex3Mod"
<220>
   <221> misc_feature
   <222> (2)..(2)
   <223> n is a, c, g, or t
<220>
   <221> misc_feature
   <222> (14)..(14)
   <223> n is a, c, g, or t
<400> 2
   tngcccagtt rttnccdgc 19
<210> 3
   <211> 21
   <212> DNA
   <213> artificial sequence
<220>
   <223> Primer reverse "Arex3-1Mod"
<220>
   <221> misc_feature
   <222> (15)..(15)
   <223> n is a, c, g, or t
<400> 3
   gtrtagtrvc cyttngccca g 21

## Description

The present invention falls within the field of foodstuff analysis.

More particularly, the invention concerns a method and a related kit for identifying the animal species in the derived material of animal origin and in the foodstuffs containing it.

The identification of the animal species in the products derived therefrom is particularly effective when it relies on the detection of diagnostic DNA sequences, the latter not being susceptible, unlike proteins or metabolites, to changes elicited by external factors related to the production chain that goes from animal feeding up to the various steps of the industrial process.

The current techniques of species identification, which comprise the detection of diagnostic DNA regions, are based on two main approaches, which may optionally be used in combination with one another: 1) detection, through PCR (Polymerase Chain Reaction), of species-specific amplification profiles of individual DNA sequences; 2) analysis of the nucleotide sequences of the same amplification products or of other DNA portions considered to carry species-specific polymorphisms.

WO2008/056325 discloses a method for molecular detection and identification of animal species characterized in that polymorphic insertion and deletion of nucleotides are analysed in mitochondial DNA 12s and 16s ribosomal RNA genes.

Amplification by a single PCR reaction requires the detection of oligonucleotide primer pairs (primers) which allow either for a selective multiplication of species-specific DNA sequences (all or nothing reaction) or the obtainment of amplified fragments the size of which may vary among species (species-specific markers). The detection and interspecific coverage levels of the PCR approach alone, based on a single reaction, are somewhat limited. In some cases, the interspecific resolution can be increased by setting up several parallel PCR reactions, using different primers that produce a higher number of markers, or by setting up multiplex PCR reactions, wherein primer pairs designed to amplify DNA fragments of different sizes are added in the same reaction mixture. One variant of the latter approach is represented by a simultaneous amplification, from the same genome, of one or more DNA fragments which may correspond to short sequence repeat families, i.e. microsatellite sequences also designed as SSR (Simple Sequence Repeat), or of fragments that are generated with a pre-digestion of the genomic DNA by restriction enzymes, a technique called AFLP (Amplified Fragment Length Polymorphism). In the first case (SSR), the resolution level is nearly exclusively intraspecific, whereas in the second case (AFLP), the resolution level is also interspecific. In terms of traceability of the species and, even more, of single individuals of the species, the SSR and AFLP markers may optionally cooperate in defining, together with SNP (single nucleotide polymorphism)-related sequence data and INDEL (insertions/deletions), a genomic profile of the animal which may be later found in the foodstuffs derived therefrom.

The use of DNA sequencing of the PCR-amplified products or of genomic regions considered to be highly polymorphic, decidedly improved the coverage and interspecific resolution levels in the analysis of food traceability. Owing to its inherent features, DNA sequencing relies on detection of sequence polymorphisms sometimes restricted to single nucleotide base substitutions, as in the case of SNPs. Although the target sequences may be the most varied, the current trend is to privilege, if not even to restrict, the sequencing to genes present in mitochondrial DNA, at least for animal organisms. The 5' portion of the *cox1* mitochondrial gene, which encodes for the cytochrome C oxidase subunit 1, is currently used as an almost universal barcode in animal multicellular eukaryotic organisms. It is through its sequencing that one also tries to recognize the identity of the animal species in the products derived therefrom, such as consumer meats and fish. In such a context, each species is thus classified on the basis of the sequence features of its *cox1* gene. For such sequences, an international deposit site exists which is called BOLD (Barcoding Of Life Depository).

At present, the application of this analysis tool to meat and fish, when present in non-complex food matrixes, has generated dissimilar results. Individual fish species may be identified, with a good reliability and coverage, also thanks to the presence of an extensive reference databank that on its own guarantees an interspecific coverage of 26%. For the remaining 74% of species, as well as for the new species introduced on the market, for the local varieties and also for other fish products, such as gastropods, crabs, lobsters, holothurians, there are problems of a better identification. As regards meat animals, the identification problem is greater, given the still low number of species in the reference databank and the many cases where the species, or their crossbreed, have the same DNA sequence as *cox1.*

Both of the above-described approaches, i.e. PCR amplification with individual primer pairs and DNA sequencing, as well as several combinations and variations thereof, share a conceptual limitation that turns into a major application limit. In the target DNA sample, the known methodologies rely on the examination of the presence or absence of a predetermined region or sequence. In fact, the prior art methodologies require a previous knowledge, based on which specific primers can be designed and used, which can trigger an equally specific amplification reaction or a sequencing reaction the resolution specificity of which requires the consultation of a reference databank.

Therefore, there is a need for a method for identifying the animal species to which the material of animal origin that is present in a food product belongs, which overcomes the disadvantages of the prior art.

Such a need has now been met by the present inventors who determined, with their own studies, that the selective amplification of the region corresponding to the third intron of the genes of the animal beta tubulin family by a pair of degenerated primers that are effective for all animal species, allows to obtain a multiple amplification product having features that make it associable with the species present in the food sample analyzed.

The present invention, as described in greater detail hereinafter, is thus based on the identification of the species *a posteriori* by using a primer common to all animal species. This brings along many advantages: 1) one single PCR reaction is required, which is triggered by a pair of degenerated primers that, as demonstrated by the experiments carried out by the present inventors, are at least effective for all fish species and for all meat animals; 2) the PCR reaction generates a pattern of amplification bands suitable to be used as a barcode that is immediately associable with the species; 3) a specific reference databank consisting of barcodes easily obtainable from any animal species can be used for identifying the species; 4) there is a tangible possibility of recognizing the individual ingredients present in a mixture; 5) there is a tangible possibility of detecting non-declared species that may occur in the analyzed product by virtue of contaminations or adulterations; 6) it is possible to obtain a higher resolution at the local variety, subspecies or breed level.

The European patent EP 1144691 describes the use of the tubulin gene introns as a source of genetic polymorphism useful for the traceability and genetic certification of plant species. However, the effectiveness of the animal beta tubulin gene, as a source of genetic polymorphism for identifying the species in the field of animal foodstuffs, is to be considered unexpected.

In fact, there is a considerable amino acid diversity among the different isoforms of animal and plant tubulins, which is even higher at the nucleotide level. Such a diversity derives from the fact that plant tubulins, compared to the animal ones, give rise to, in addition to the mitotic spindle, three other organizational states of the microtubules: the pre-prophase band, the fragmoplast, and the cortical system, which mark and sustain growth modes for cell division and spreading unconnected with the animal kingdom, but typical of plants. Moreover, the gene families of animal tubulins (alpha and beta) are much less than the plant ones. Furthermore, the organization of single genes differs both in the expression regulatory sequences and in the number and localization of introns, usually much shorter in plants than in animal organisms. Such a difference at the DNA level is largely represented by the different levels of gene organization and expression which distinguish animal cells from plant cells. The mitochondrial *cox1* gene itself, used as a barcode sequence in animal organisms, is not usable in plants because its nucleotide sequence is, in the latter, highly conserved among species, reason why markers of chloroplast origin are used instead. In corroboration of such actual diversity, higher plants are only shown to have two introns in the beta tubulin genes, both usable as species-discriminating markers. On the contrary, in animal organisms, the nucleotide sequences of the first two introns do not give effective amplification primers, which are only present at the third intron, which thus meets all the specificity requirements needed for its use as a diagnostic sequence.

Therefore, an object of the present invention is a method of identifying the animal species to which the material of animal origin that is present in a food product belongs, as defined in the appended claim 1.

Also a kit for identifying the animal species to which the material of animal origin that is present in a food product belongs, as defined in the appended claim 8, as well as the oligonucleotides that serve as primers, as defined in the appended claim 11, fall within the scope of the present invention.

The dependent claims define further features of the invention and form an integral part of the specification.

In a preferred embodiment of the present invention, the amplification reaction of the region of the third intron of the animal beta tubulin gene is performed by a polymerase chain reaction (PCR). The PCR is a largely known amplification technique for nucleic acids, the implementation of which is within the capabilities of one of ordinary skill in the art. The preliminary steps optionally required for genomic DNA extraction and purification from the sample to be analyzed are also feasible by conventional methodologies that fall within the abilities of one of ordinary skill in the art.

In a further preferred embodiment of the present invention, the PCR reaction is carried out by using degenerated oligonucleotide primers, i.e. mixtures of oligonucleotide primers that are similar, but not necessarily identical. In such an embodiment, the pair of degenerated oligonucleotide primers consists of a first degenerated oligonucleotide primer which is able to hybridize to the genomic DNA of the material of animal origin at an exonic region flanking, at the 5' side, the third intron of the animal beta tubulin gene and a second degenerated oligonucleotide primer which is able to hybridize to the genomic DNA of the material of animal origin at an exonic region flanking, at the 3' side, the third intron of the animal beta tubulin gene.

Degenerated oligonucleotide primers according to the present invention are the degenerated oligonucleotide primer having the sequence SEQ ID NO:1 (forward primer) and the degenerated oligonucleotide primer having the sequence SEQ ID NO: 2 or SEQ ID NO: 3 (reverse primer).
The genomic DNA amplification of the foodstuff to be analyzed provides an amplification product, which is analyzed to detect a feature that is indicative of the animal species to which said material of animal origin belongs. Said feature is preferably the pattern of the amplification bands. Methods useful to this end are for example native polyacrylamide gel electrophoresis or capillary electrophoresis. Alternatively, the feature detected is the presence of one or more species-specific DNA sequence(s). Methods useful to this end are for example a hybridization with molecular probes or a quantitative PCR. The above-mentioned processes suitable to be used in the analysis phase of the method of the present invention are all largely known and the selection and performance thereof fall within the capabilities of one of ordinary skill in the art.

The following exemplary embodiment is provided solely by way of illustration and not of limitation of the scope of the claims, as defined in the appended claims.

### EXAMPLE

### ANIMAL MATERIAL USED

*Bos taurus* (bullock), *Oryctolagus cuniculus* (rabbit), *Sus scrofa* (pig), *Equus caballus* (horse), *Ovis aries* (goat)*, *Meleagris gallopavo* (turkey), *Gallus gallus* (chicken), *Xiphias gladius* (swordfish), *Salmo salar* (salmon)**.
* cells pelleted from raw goat milk
**lyophilized commercial DNA extract

Alternatively, meat purchased from the common commercial channels are used as the starting material.

### METHODS

An analysis method according to the invention was carried out, comprising the following steps:
1) Genomic DNA (gDNA) extraction from the samples.
2) PCR amplification of the intron sequences of the animal beta tubulin genes.
3) Electrophoresis analysis: separation of the different fragments on acrylamide gel, obtained by amplification of the gDNA extracted.
4) Assessment of the polymorphic profiles obtained.
5) Isolation from the acrylamide gel of a few intronic fragments obtained from the amplification profiles.
6) Cloning and sequencing of the above-indicated intronic fragments in order to confirm the identity of the isolated bands as beta tubulin isotypes.

The used degenerated primers were designed on alignments of beta tubulin gene sequences deposited at the NCBI GenBank databank.

The following degenerated primers were used:
Forward primer ("Afex3Mod"):
   5'-GAYYTDGARCCNGGNACNATGG-3' (SEQ ID NO:1)
Reverse primer ("Arex3Mod"):
   5'-TNGCCCAGTTRTTNCCDGC-3' (SEQ ID NO:2)
Reverse primer ("Arex3-1Mod"):
   5'-GTRTAGTRVCCYTTNGCCCAG-3' (SEQ ID NO:3)
   degenerations: A/G = R; C/T = Y; A/G/T = D; A/C/G = V; C/G/A/T = N

The above-described degenerated primers were used in alternation in the two following combinations:
Primer pair 1: Afex3Mod/ Arex3Mod
Primer pair 2: Afex3Mod/ Arex3-1Mod

The primers were designed on the exon regions flanking, at the 5' and 3' ends, the third intron of the animal beta tubulin genes at the distances indicated below, expressed as nucleotide base pairs, with regard to the position of the first ATG codon useful for translation:
200 base pairs from the ATG for the forward primer "Afex3Mod";
289 base pairs from the ATG for the reverse primer "Arex3Mod";
300 base pairs from the ATG for the reverse primer "Arex3-1Mod".

The insertion site for the intron is found at a distance of:
78 base pairs from the 5' end of the forward primer "Afex3Mod";
29 base pairs from the 3' end of the reverse primer "Arex3Mod";
42 base pairs from the 3' end of the reverse primer "Arex3-1Mod".

The degenerated primers used are capable of amplifying the third intron in the beta tubulin genes, which has various sizes depending on the animal species. Besides the intron, also the two short exon sequences that flank it at both the 5' and 3' ends, are amplified, which are a total of 107 or 120 base pairs in length depending on the two primer combinations used.

The genomic DNA was extracted by using a commercial kit, Nucleospin food (Machery-Naghel), starting with 200 mg of animal tissue frozen in liquid nitrogen and then ground by using a mortar or with a cell pellet collected from 50 ml of raw goat milk. The quantity and quality of the extracted DNA were assessed by spectrophotometric readings and electrophoretic runs on agarose gels.

The intronic sequences of the β-tubulin genes were amplified selectively by PCR starting with 100 and/or 400 ng of gDNA using, in alternation, the two different combinations of forward and reverse primers quoted above.

The following amplification protocol was used:
1. 3 min at 94°C (initial denaturation)
   Repeated for 14 cycles:
2. 30 sec at 94°C (denaturation)
3. 45 sec at 67°C (annealing of primers to DNA at the first amplification round)
4. 2 min at 72°C (extension)
   at each cycle, the annealing temperature decreases by 0,7°C
   Repeated for 20 cycles:
5. 30 sec at 94°C (denaturation)
6. 45 sec at 57°C (annealing of primers to DNA)
7. 2 min at 72°C (extension)
   For 1 cycle:
8. 30 min at 72°C (final extension)

Maintenance at 10°C until the next step.

For each sample analyzed, the PCR reaction was performed in a volume of 30µl containing, in addition to the template DNA, the following reagents the final concentration of which is indicated:
1U of Taq Polymerase
1x dilution of the buffer associated therewith (10 mM tris-HCl pH8.8, 50 mM KCl)
3 mM of Mg²⁺
0.2 mM of an equimolar mixture of deoxynucleotides (dATP, dCTP, dGTP, dTTP)
1µM oligonucleotide primers (primer pairs Afex3Mod/ Arex3Mod or Afex3Mod/ Arex3-1Mod).

The same reaction was carried out without the template DNA to assess the possible presence of false positives. The reproducibility of the amplification reactions was verified performing the PCR reactions at least 3 times.

The quality of the amplification was assessed by electrophoresis. One aliquot of the amplification products obtained from the PCR reactions is preliminarily loaded on a 2% w/v agarose gel, to check the success of the reaction. The DNA is visualized by staining with ethidium bromide at a concentration of 100 ng/ml.

Successively, an electrophoresis was performed on a non-denaturing 6% w/v acrylamide gel, using the sequencing gel apparatus Sequi-Gen (BIO-RAD).

At the end of the electrophoresis, following the fixation of the DNA fragments, the same are stained with a 0.15% silver nitrate solution (according to the protocol reported by Bassam et al. Anal Biochem. 1991 Jul; 196(1):80-3). The gel fixed on a glass plate was left to dry and the image of the polymorphic band profile was acquired by scanning and converted into an image file for further processing.

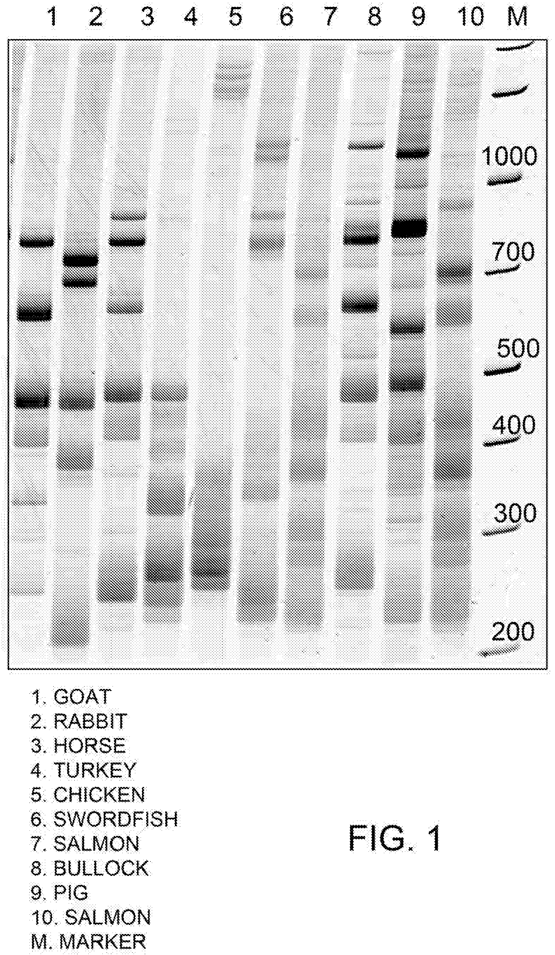

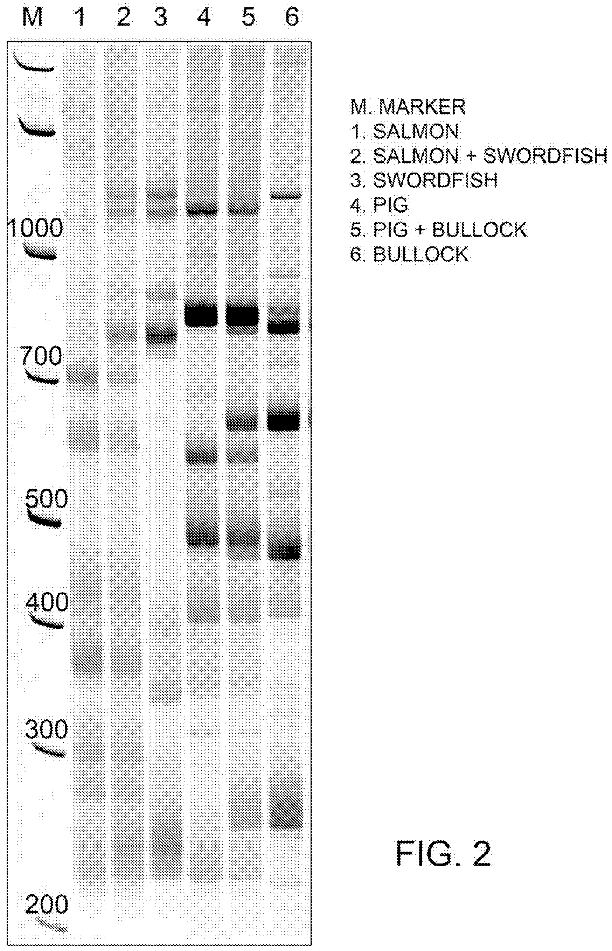

The obtained results are illustrated in Figures 1 and 2.

Figure 1 shows the image of a polyacrylamide gel on which 2 to 4 µl of the amplification products of the third intron of the beta tubulins were loaded, which had been obtained by PCR from 200 ng of genomic DNA of animal origin using the primer pair Afex3Mod/ Arex3-1Mod. In the figure, the nine different species are characterized by a species-specific banding profile having a size comprised between 200 and 1000 base pairs. Samples 7 and 10 represent two different amplifications obtained from salmon gDNA.

Figure 2 shows the amplification products obtained from 200 ng of gDNA from individual animal species (salmon, swordfish, pig, and bullock) and from mixtures thereof (salmon plus swordfish and pig plus bullock) with the primer pair Afex3Mod/Arex3-1Mod. As inferred from the figure, the four single species, salmon, swordfish, bullock, pig (lanes 1, 3, 4, 6) are characterized by a unique and species-specific banding. The polymorphic band profile characterizing the single species is also perfectly recognizable in the respective mixtures (lanes 2 and 5) where it is possible to distinguish the concurrent presence of salmon and swordfish (lane 2) and pig and bullock (lane 5).

## Claims

1. A method of identifying the animal species to which the material of animal origin that is present in a food product belongs, comprising the steps of:
1) providing a food product comprising material of animal origin;
2) selectively amplifying at least one region of the genomic DNA of said material of animal origin, thereby obtaining an amplification product having a feature which is indicative of the animal species to which said material of animal origin belongs;
3) analyzing said feature of the amplification product, thereby identifying the animal species to which the material of animal origin belongs, **characterized in that** said at least one region of the genomic DNA is a region of the third intron of the genes encoding for animal β-tubulin.

2. The method according to claim 1, wherein said step of selectively amplifying at least one region of the genomic DNA of the material of animal origin is performed by Polymerase Chain Reaction (PCR).

3. The method according to claim 2, wherein said Polymerase Chain Reaction (PCR) is performed by the use of a pair of degenerated oligonucleotide primers consisting of a first degenerated oligonucleotide primer which is able to hybridize to the genomic DNA of the material of animal origin at an exonic region flanking, at the 5' side, the third intron of the genes encoding for animal β-tubulin and a second degenerated oligonucleotide primer which is able to hybridize to the genomic DNA of the material of animal origin at an exonic region flanking, at the 3' side, the third intron of the genes encoding for animal β-tubulin.

4. The method according to claim 3, wherein said first degenerated oligonucleotide primer is SEQ ID NO:1.

5. The method according to claim 3 or 4, wherein said second degenerated oligonucleotide primer is SEQ ID NO:2 or SEQ ID NO:3.

6. The method according to any of claims 1 to 5, wherein said feature of the amplification product of the genomic DNA of the material of animal origin is the pattern of the amplification bands.

7. The method according to any of claims 1 to 5, wherein said step of analyzing said feature of the amplification product of the genomic DNA of the material of animal origin is performed by polyacrylamide gel electrophoresis, capillary electrophoresis, hybridization or quantitative PCR.

8. A kit for identifying the animal species to which the material of animal origin that is present in a food product belongs, comprising a pair of degenerated oligonucleotide primers consisting of a first degenerated oligonucleotide primer which is able to hybridize to the genomic DNA of the material of animal origin at an exonic region flanking, at the 5' side, the third intron of the genes encoding for animal β-tubulin and a second degenerated oligonucleotide primer which is able to hybridize to the genomic DNA of the material of animal origin at an exonic region flanking, at the 3' side, the third intron of the genes encoding for animal β-tubulin, said pair of degenerated oligonucleotide primers being capable of amplifying a region of the third intron of the genes encoding for animal β-tubulin.

9. The kit according to claim 8, wherein the first degenerated oligonucleotide primer is SEQ ID NO:1.

10. The kit according to claim 8 or 9, wherein the second degenerated oligonucleotide primer is SEQ ID NO:2 or SEQ ID NO:3.

11. A primer pair consisting of the degenerated oligonucleotides of SEQ ID NO:1 and SEQ ID NO:2 or of the degenerated oligonucleotides of SEQ ID NO:1 and SEQ ID NO:3.

## Patentansprüche

1. Verfahren zum Identifizieren der Tierarten, zu denen ein Material tierischer Herkunft gehört, das in einem Nahrungsmittelprodukt vorliegt, umfassend die Schritte:
1) Bereitstellen eines Nahrungsmittelprodukts, das ein Material tierischer Herkunft umfasst;
2) selektiv Amplifizieren mindestens einer Region der genomischen DNA dieses Materials tierischer Herkunft, wodurch ein Amplifizierungsprodukt mit einem Merkmal erhalten wird, das auf die Tierart hinweist, zu der das Material tierischer Herkunft gehört;
3) Analysieren des Merkmals des Amplifizierungsprodukts, wodurch die Tierart identifiziert wird, zu der das Material tierischer Herkunft gehört, **dadurch gekennzeichnet, dass** die mindestens eine Region der genomischen DNA eine Region des dritten Introns der Gene ist, die tierisches β-Tubulin kodieren.

2. Verfahren nach Anspruch 1, bei dem der Schritt des selektiven Amplifizierens mindestens einer Region der genomischen DNA des Materials tierischer Herkunft durch eine Polymerasekettenreaktion (PCR) durchgeführt wird.

3. Verfahren nach Anspruch 2, bei dem die Polymerasekettenreaktion (PCR) durch die Verwendung eines Paars von degenerierten Oligonukleotidprimern durchgeführt wird, das aus einem ersten degenerierten Oligonukleotidprimer, der an die genomische DNA des Materials tierischer Herkunft an einer exonischen Region hybridisieren kann, die, an der 5'-Stelle, das dritte Intron der Gene flankiert, die tierisches β-Tubulin kodieren, und einem zweiten degenerierten Oligonukleotidprimer besteht, der an die genomische DNA des Materials tierischer Herkunft an einer exonischen Region hybridisieren kann, die, an der 3'-Stelle, das dritte Intron der Gene flankiert, die tierisches β-Tubulin kodieren.

4. Verfahren nach Anspruch 3, bei dem der erste degenerierte Oligonukleotidprimer SEQ ID NO:1 ist.

5. Verfahren nach Anspruch 3 oder 4, bei dem der zweite degenerierte Oligonukleotidprimer SEQ ID NO:2 oder SEQ ID NO:3 ist.

6. Verfahren nach einem der Ansprüche 1 bis 5, bei dem das Merkmal des Amplifizierungsprodukts der genomischen DNA des Materials tierischer Herkunft die Struktur der Amplifizierungsbanden ist.

7. Verfahren nach einem der Ansprüche 1 bis 5, bei dem der Schritt des Analysierens des Merkmals des Amplifizierungsprodukts der genomischen DNA des Materials tierischer Herkunft durch eine Polyacrylamidgelelektrophorese, Kapillarelektrophorese, Hybridisierung oder quantitative PCR durchgeführt wird.

8. Kit zum Identifizieren der Tierarten, zu denen ein Material tierischer Herkunft gehört, das in einem Nahrungsmittelprodukt vorliegt, umfassend ein Paar von degenerierten Oligonukleotidprimern, das aus einem ersten degenerierten Oligonukleotidprimer, der an die genomische DNA des Materials tierischer Herkunft an einer exonischen Region hybridisieren kann, die, an der 5'-Stelle, das dritte Intron der Gene flankiert, die tierisches β-Tubulin kodieren, und einem zweiten degenerierten Oligonukleotidprimer besteht, der an die genomische DNA des Materials tierischer Herkunft an einer exonischen Region hybridisieren kann, die, an der 3'-Stelle, das dritte Intron der Gene flankiert, die tierisches β-Tubulin kodieren, wobei das Paar von degenerierten Oligonukleotidprimern eine Region des dritten Introns der Gene, die tierisches β-Tubulin kodieren, amplifizieren kann.

9. Kit nach Anspruch 8, bei dem der erste degenerierte Oligonukleotidprimer SEQ ID NO:1 ist.

10. Kit nach Anspruch 8 oder 9, bei dem der zweite degenerierte Oligonukleotidprimer SEQ ID NO:2 oder SEQ ID NO:3 ist.

11. Primarpaar, das aus den degenerierten Oligonukleotiden von SEQ ID NO:1 und SEQ ID NO:2 oder den degenerierten Oligonukleotiden von SEQ ID NO:1 und SEQ ID NO:3 besteht.

## Revendications

1. Procédé d'identification de l'espèce animale à laquelle appartient la matière d'origine animale qui est présente dans un produit alimentaire, comprenant les étapes :
1) de fourniture d'un produit alimentaire comprenant de la matière d'origine animale ;
2) d'amplification sélective d'au moins une région de l'ADN génomique de ladite matière d'origine animale, obtenant ainsi un produit d'amplification ayant une particularité qui est indicative de l'espèce animale à laquelle appartient ladite matière d'origine animale ;
3) d'analyse de ladite particularité du produit d'amplification, identifiant ainsi l'espèce animale à laquelle appartient la matière d'origine animale, **caractérisé en ce que** ladite au moins une région de l'ADN génomique est une région du troisième intron des gènes codant pour la β-tubuline animale.

2. Procédé selon la revendication 1, dans lequel ladite étape d'amplification sélective d'au moins une région de l'ADN génomique de la matière d'origine animale est réalisée par réaction en chaîne par polymérase (PCR).

3. Procédé selon la revendication 2, dans lequel ladite réaction en chaîne par polymérase (PCR) est réalisée par l'utilisation d'une paire d'amorces d'oligonucléotides dégénérés consistant en une première amorce d'oligonucléotide dégénéré qui est capable de s'hybrider à l'ADN génomique de la matière d'origine animale au niveau d'une région exonique flanquant, au niveau du côté 5', le troisième intron des gènes codant pour la β-tubuline animale et une seconde amorce d'oligonucléotide dégénéré capable de s'hybrider à l'ADN génomique de la matière d'origine animale au niveau d'une région exonique flanquant, au niveau du côté 3', le troisième intron des gènes codant pour la β-tubuline animale.

4. Procédé selon la revendication 3, dans lequel ladite première amorce d'oligonucléotide dégénéré est SEQ ID n° 1.

5. Procédé selon la revendication 3 ou 4, dans lequel ladite seconde amorce d'oligonucléotide dégénéré est SEQ ID n° 2 ou SEQ ID n° 3.

6. Procédé selon l'une quelconque des revendications 1 à 5, dans lequel ladite particularité du produit d'amplification de l'ADN génomique de la matière d'origine animale est le modèle des bandes d'amplification.

7. Procédé selon l'une quelconque des revendications 1 à 5, dans lequel ladite étape d'analyse de ladite particularité du produit d'amplification de l'ADN génomique de la matière d'origine animale est réalisée par électrophorèse sur gel de polyacrylamide, électrophorèse capillaire, hybridation ou PCR quantitative.

8. Kit pour identifier l'espèce animale à laquelle appartient la matière d'origine animale qui est présente dans un produit alimentaire, comprenant une paire d'amorces d'oligonucléotides dégénérés consistant en une première amorce d'oligonucléotide dégénéré qui est capable de s'hybrider à l'ADN génomique de la matière d'origine animale au niveau d'une région exonique flanquant, au niveau du côté 5', le troisième intron des gènes codant pour la β-tubuline animale et une seconde amorce d'oligonucléotide dégénéré qui est capable de s'hybrider à l'ADN génomique de la matière d'origine animale au niveau d'une région exonique flanquant, au niveau du côté 3', le troisième intron des gènes codant pour la β-tubuline animale, ladite paire d'amorce d'oligonucléotide dégénéré étant capable d'amplifier une région du troisième intron des gènes codant pour la β-tubuline animale.

9. Kit selon la revendication 8, dans lequel la première amorce d'oligonucléotide dégénéré est SEQ ID n° 1.

10. Kit selon la revendication 8 ou 9, dans lequel la seconde amorce d'oligonucléotide dégénéré est SEQ ID n° 2 ou SEQ ID n° 3.

11. Paire d'amorces consistant en les oligonucléotides dégénérés de SEQ ID n° 1 et SEQ ID n° 2 ou les oligonucléotides dégénérés de SEQ ID n° 1 et SEQ ID n° 3.
